(19) Europäisches Patentamt

European Patent Office

Office européen des brevets

(11) Veröffentlichungsnummer : **0 478 532 A1**

(12) **EUROPÄISCHE PATENTANMELDUNG**

(21) Anmeldenummer : 91890194.3

(22) Anmeldetag : 02.09.91

(51) Int. Cl.$^5$ : **A61F 2/30**

(30) Priorität : **07.09.90 AT 1829/90**

(43) Veröffentlichungstag der Anmeldung :
**01.04.92 Patentblatt 92/14**

(84) Benannte Vertragsstaaten :
**BE CH DE DK ES FR GB GR IT LI LU NL SE**

(71) Anmelder : **David, Thomas, Dr.
Margaretenstrasse 2-4
A-1040 Wien (AT)**

(72) Erfinder : **David, Thomas, Dr.
Margaretenstrasse 2-4
A-1040 Wien (AT)**

(74) Vertreter : **Collin, Hans, Dipl.-Ing. Dr. et al
Patentanwälte Dipl.-Ing. Dr. Hans Collin
Dipl.-Ing. Erwin Buresch Dipl.-Ing.Armin Häupl
Mariahilferstrasse 50
A-1070 Wien (AT)**

(54) **Endoprothese.**

(57)    Vorgeschlagen wird eine Endoprothese mit einem kräfteaufnehmenden Metallteil, insbesondere Titanteil, bei der am Metallteil (1)zumindest abschnittsweise eine an den Metallteil angeformte, insbesondere den Metallteil durchgreifende Auflage und/oder Einlage (2) aus osteokonduktivem bzw. osteoinduktivem Implantatmaterial vorgesehen ist.

Fig. 3

EP 0 478 532 A1

Die Erfindung betrifft eine Endoprothese mit einem kräfteaufnehmenden Metallteil.

Endoprothesen aus Metall - insbesondere aus Titan oder Implantatstahl, die auch beschichtet sein können - haben sich in großem Maße durchgesetzt; das Hauptproblem ist dabei seit jeher deren dauerhafte Fixation. In diesem Zusammenhang ist vor allem wesentlich, daß sich das bisherige Einzementieren von Endoprothesen nicht bewährt hat, ebenso nicht das Beschichten von Endoprothesen mit Hydroxyapatit. Es zeigte sich, daß bestimmte Beschichtungen im Körper Abwehrreaktionen induzieren; die Prothesen lockern sich nach einiger Zeit.

Das heutige Ziel der Endoprothetik (vor allem bei Patienten zwischen 30 und 65 Jahren) liegt in der zementfrei implantierten Metallprothese und demgemäß in deren einwandfreier primärer Fixierung.

Hiezu hat man schon versucht, Metallendoprothesen zu entwickeln, die sich im Einsatzknochen durch den Einschlagdruck verkeilen, wobei auch schon fräserartig wirkende Verzahnungen an Metallendoprothesen bekannt sind.

Es ist jedoch so, daß die medizinischen Gegebenheiten eine Anwendung derartiger Endoprothesen, die verformende oder gar abtragende Wirkungen an der Innenseite des Einsatzknochens haben, kontraindizieren, insbesondere wenn man in Rechnung zieht, daß Endoprothesen vor allem im fortgeschrittenen Lebensalter nötig werden, wo ein natürlicher Altersabbau eine mehr oder weniger starke Schwächung der tragenden Kortikalisschicht hervorrufen kann.

Eine, vor allem femorale, Endoprothese sollte über deren ganze Länge gleichmäßig, vor allem aber proximal, möglichst ohne ungleichmäßige lokale Druckverteilung (keine Spitzendrücke nach außen) im Einsatzknochen fixiert werden können.

Es sind bereits Implantatmaterialien als Knochenreparaturstoffe bekannt geworden, die biokompatibel sind und, soferne sie in eine gesunde körperversorgte Umgebung eingesetzt werden, von osteogenen Zellen durchwachsen werden können. Diese Materialien werden osteokonduktive Implantatmaterialien genannt. Hier sind die sogenannten BOP-Materialien bekannt, die in jeder körperlichen Form herstellbar sind aber auch intraoperativ noch anatomisch anpaßbar sind und neben dem Durchwachsenwerden durch osteogene Zellen den Vorteil haben, biodegradabel zu sein. BOP ist die internationale Abkürzung für Biocompatible (and reconstructive) Othopaedic Polymer und ist ein Kopolymer aus N-Polyvinylpyrrolidon und Methylmethacrylat, gegebenenfalls mit Füllstoffen, wie Polyamidfasern und Ca-Glukonat. Diese Materialien werden von Knochengewebe durchwachsen und resorbiert; die Osteifikation dauert etwa 5 Monate bis 1,5 Jahre.

Eine weitere Gruppe derartiger Materialien sind konduktive und darüber hinaus induktive Implantatmaterialien, die nicht nur eine konduktive Matrix, sondern einen osteogenen Faktor enthalten, der das Einwachsen von Knochengewebe (mit oder ohne einer intermediären Knorpelbildung) induziert. Ein derartiges Material auf Kollagenbasis ist z.B. in der EP-A 0 182 483 beschrieben und weist als Besonderheit neben dem osteoinduktiven Faktor (OF) eine Matrix auf, die einen bestimmten Anteil an nichtfibrillarem Kollagen, insbesondere Atelopeptidkollagen, enthält. In der Matrix können als Hauptanteil z.B. Kollagenpulver und/oder Hydroxyapatit vorliegen.

Es ist bereits vorgeschlagen worden, derartige Implantatmaterialien in Kombination mit Endoprothesen zum Zweck einzusetzen, Hohlräume zwischen der Prothese und dem umgebenden Knochen auszufüllen.

Dieses Verfahren weist den Nachteil auf, daß das Implantatmaterial praktisch erst nach dem Setzen der Prothese einbringbar ist, somit nicht überall dort, wo es benötigt würde, und überdies, daß damit keine form-und kraftschlüssige schlüssige Verbindung zwischen dem vorhandenen Knochen, dem neu gebildeten Knochenmaterial und der Endoprothese herstellbar ist und daher ist eine übungs- und belastungsstabile Primärfixation nicht möglich.

Es bestand daher die medizinische Aufgabe, diese Mängel zu beseitigen. Diese Aufgabe wird erfindungsgemäß dadurch gelöst, daß das biokompatible, bioinerte bzw. osteoinduktive Material einen stabilen, vorausberechneten, vorausgeformten, aber daneben den anatomischen individuellen Bedürfnissen anpaßbaren Bestandteil der Endoprothese bildet, der an deren vorher berechneten Metallteil form- und kraftschlüssig angeformt ist.

Vorher berechnet und angeformt bedeutet in diesem Zusammenhang, daß die erfindungsgemäßen Endoprothesen eine stabile Einheit aus Metallteil und Implantatmaterial darstellen, mit dem Zweck, daß das einwachsende Knochenmaterial einen form- und kraftschlüssigen Verbund mit dem Metallteil bildet.

Demgemäß ist eine erfindungsgemäße Endoprothese vor allem dadurch gekennzeichnet, daß am Metallteil zumindest abschnittsweise eine an den Metallteil angeformte, insbesondere den Metallteil durchgreifende Auflage und/oder Einlage aus osteokonduktivem bzw. osteoinduktivem Implantatmaterial vorgesehen ist. Insbesondere weisen dabei Auflage und/oder Einlage sowie Metallteil eine korrespondierende Verzahnung auf, die auch durch Querbohrungen gebildet sein kann, in die osteoinduktives Material eingesetzt ist.

Insbesondere kann die Auflage und/oder Einlage als, mit dem Metallteil form- bzw. kraftschlüssig verbundene, insbesondere mehrteilige, Schale, welche teilweise anatomisch form- und kraftschlüssig an den Knochen angeformt, anderseits form- und kraftschlüssig durch Verzahnung mit dem Metallteil, verbunden ist, oder Hülse vorgesehen sein.

Ein besonders wichtiges Kennzeichen der Erfindung ist auch, daß dem variablen Metallteil-System Auflagensätze und/oder Einlagensätze aber auch Schalen und/oder Hülsensätze in entsprechender Anzahl zugeordnet sind. Damit kann insbesondere dem altersbedingten Knochenabbau Rechnung getragen werden. Insbesondere sind die Auflagensätze so ausgebildet, daß beim Setzen der Prothese an Ort und Stelle Implantatmaterial abgetragen werden kann, um einen gleichmäßigen satten Sitz im Knochen zu erzielen.

Selbstverständlich ist auch eine partielle oder totale Oberflächenbehandlung am Metallteil möglich, insbesondere zur Erhöhung der Oberflächenporosität, oder als Beschichtung zum Zwecke der besseren Kraftübertragung.

Bei der Ausbildung einer erfindungsgemäßen Endoprothese als Femurprothese ist bevorzugt, daß der Prothesenschaft als Flachprofil, teilweise anatomisch form- und kraftschlüssig oder proximal eventuell als Rund-(Keil-) Profil mit sich verjüngender Verzahnung, bezüglich der Form und kraftschlüssig angepaßt, vor allem proximal mit lateral angeorneten, vorzugsweise das Schaftprofil anatomisch ausfüllenden Platten aus Implantatmaterial, ausgebildet ist. Ein Flachprofil des Schafts gestattet bei einer Femurprothese eine satte Anlage an der Innenseite sowohl des craniolateralen als auch des caudomedialen Femurpfeilers, wo die größte Cortikalisstärke vorhanden ist und daher die günstigste Kraftübertragungsfläche besteht.

Eine weitere vorteilhafte Ausführungsform einer Femurprothese ist dadurch gekennzeichnet, daß der Prothesenschaft in seinem mittleren und/oder distalen Bereich bis zum Ende gespalten ist und das Implantatmaterial als Einlageblock in einer im wesentlichen Dreieckprofil aufweisenden Durchbrechung im proximalen Bereich des Prothesenschaftes vorgesehen ist, gegebenenfalls mit einem zentral im Schaft längsverschieblichen stab- oder rohrförmigen Implantatmaterialeinsatz, der im distalen Bereich unter Umständen weit über der Prothesenspitze hinaus bis zur distalen Femur-Diaphysis reichen kann. Beim Setzen der Prothese verschiebt sich der rohr- oder stabförmige Bereich des Inplantatmaterials nach proximal; am proximalen Schaftende herausgeschobenes Material wird anschließend abgestemmt oder abgefräst.

Weiterhin kann im Prothesenschaft im Bereich des Trochanter maior mindestens eine Querbohrung zur Muskelfixierung vorgesehen sein.

Weiterhin ist zum Setzen der Femurprothese bevorzugt, daß im Proximalbereich des Prothesenschaftes eine Gewindebohrung in Schaftrichtung zum Einschrauben des Prothesensetzkopfes vorgesehen ist. Die Prothese kann dadurch auf einen optimalen proximalen Sitz (unter Berücksichtigung des Abduktion- und Anteversionwinkels) exakt eingeschlagen werden; anschließend wird das Prothesensetzwerkzeug wieder abgeschraubt.

Weiterhin ist vorgesehen, daß die erfindungsgemäßen Prothesen (aus Metall und BOP oder auch anderen Materialien bestehend) auch an die individuellen anatomischen Besonderheiten des jeweiligen Knochens, vorzugsweise Oberschenkelknochens und Gelenks des Patienten anpaßbar sind; so z.B. verschiedene Größen, verschiedene Prothesenhals- und Prothesenschaftwinkel, beschichtete Metall- oder Implantatteilkragen, aufsetzbare Gelenkskopfhälse mit verschiedenen Längen, Winkelstellungen (u.a. für links oder rechts), Köpfen etc.

Zu diesem Zweck ist erfindungsgemäß z.B. eine entsprechende Ausnehmung im Proximalbereich des Metallteils einer Femurprothese und ein entsprechendes Sortiment von Implantatteilen, Prothesenkrägen, Hälsen und/oder Köpfen und vorgeformte Implantatmaterialblöcke vorgesehen.

Die Erfindung wird im folgenden anhand von Beispielen von Femurprothesen unter Bezugnahme auf die Zeichnung näher beschrieben, in der schematische Seitenansichten dargestellt sind und in der gleiche Bauteile gleiche Bezugzeichen tragen.

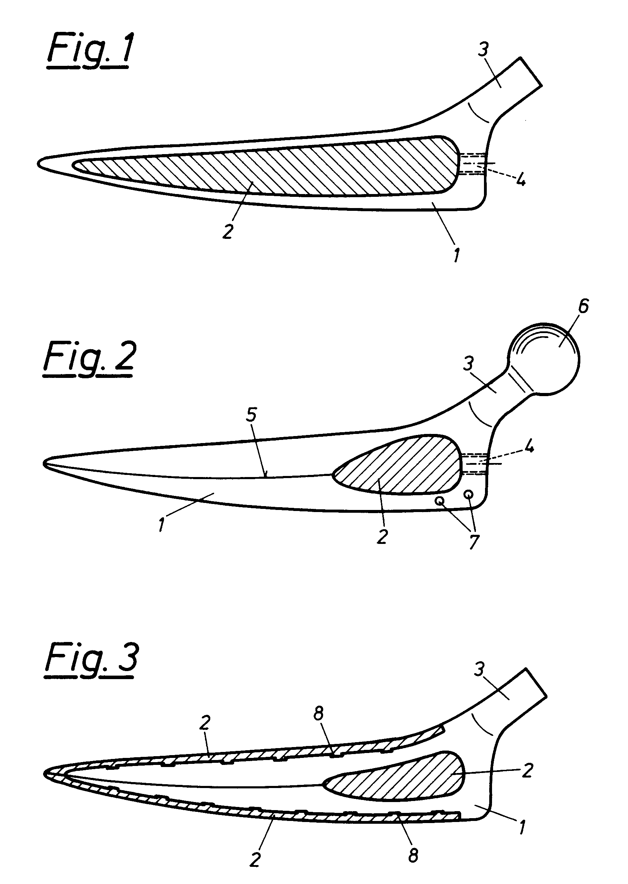

Gemäß Fig. 1 ist der Prothesenschaft 1 ein Flachprofil mit einer Durchbrechung, in die Implantatmaterial 2 als Faserbündelblock oder Platte eingesetzt ist. Der Gelenkshals ist mit 3 bezeichnet; im Proximalbereich ist eine Gewindebohrung 4 zum Anschrauben des Setzwerkzeugs zu erkennen.

Der Vorteil des Flachprofils oder proximal verzahnten Rundkeilprofils, mit mittleren und distalen Flachprofils mit Querbohrungen ist die unmittelbare primäre Fixation und die Stabilisation gegen Verdrehung.

In Fig. 2 erkennt man, daß der Prothesenschaft 1 zu seinem distalen Ende hin gespalten ist, wobei mindestens ein Schlitz 5, vorzugsweise jedoch zwei im rechten Winkel aufeinander stehende Schlitze 5 vorgesehen sind und weiterhin eine zentrale Längsbohrung in diesem (mittleren und distalen) Bereich vorgesehen sein kann, in die ein Faserbündelstab aus Implantatmaterial (auch weit über die Prothesenspitze hinaus) eingesetzt werden kann. Der Metallteil kann im gesamten Bereich querbohrungen aufweisen, die auch mit Implantatmaterial gefüllt werden können. Der Gelenkskopf ist mit 6 bezeichnet; im Bereich des Trochanter maior sind querbohrungen 7 zur Muskelfixierung angebracht.

Bei der Ausführungsform nach Fig. 3 ist das Implantatmaterial 2 wie eine Schale oder Hülse auch von außen an den Prothesenschaft 1 angeformt und mit ihm bei 8 verzahnt. Man erkennt, daß das von außen angeformte Implantatmaterial zur anatomischen Anpassung lokal abtragbar ist.

Die Implantatmaterialblöcke oder Schalenteile können anatomisch an einer oder mehreren Seiten form- und kraftschlüssig vorgeformt sein.

Die Implantatmaterialblöcke oder Schalenteile können an einer oder mehreren Seiten gleichzeitig verzahnt zur form- und kraftschlüssigen Verankerung mit dem Metallteil vorgefertigt werden.

Beide miteinander form- und kraftschlüssig verbundenen Prothesenteile werden dann als anatomisch form- und kraftschlüssige Prothese in den exakt vorpräparierten Knochen als übungs- und belastungsstabile Einheit unter Druck implantiert.

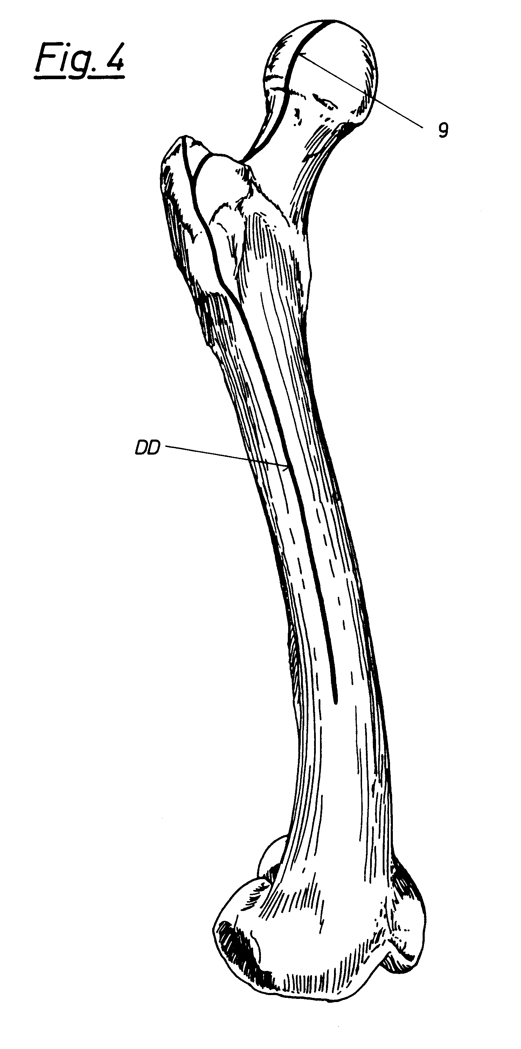

Die in den Fig. 1-3 dargestellten Femurprothesen sind in Wirklichkeit räumlich gekrümmt, zumindest in deren Schaftbereich, vorzugsweise aber auch imm Schulter- und Halsbereich. Aus Fig. 4 läßt sich diese Krümmung erkennen. Fig. 4 ist die Darstellung eines menschlichen Femur von vorne/lateral, wobei einerseits die Anteversionsebene 9 eingezeichnet ist und mit DD der Verlauf des craniolateralen Knochenpfeilers. Bevorzugt ist Schulter und Hals der Femurprothese auch gegenüber der Anteversionsebene gekrümmt, steht aber in jedem Fall im Winkel zum räumlich gekrümmten Schaft, der dem Pfeilerverlauf folgt.

## Patentansprüche

1. Endoprothese mit einem kräfteaufnehmenden Metallteil, insbesondere Titanteil, dadurch gekennzeichnet, daß am Metallteil(1)zumindest abschnittsweise eine an den Metallteil angeformte, insbesondere den Metallteil durchgreifende Auflage und/oder Einlage (2) aus oste okonduktivem bzw. osteoinduktivem Implantatmaterial vorgesehen ist.

2. Endoprothese nach Anspruch 1, dadurch gekennzeichnet, daß Auflage und/oder Einlage (2) sowie Metallteil (1) eine korrespondierende Verzahnung (8) aufweisen.

3. Endoprothese nach Anspruch 1 oder 2, dadurch gekennzeichnet, daß die Auflage und/oder Einlage (2) als,insbesondere mehrteilige, Schale vorgesehen ist.

4. Endoprothese nach einem der Ansprüche 1 bis 3, dadurch gekennzeichnet, daß dem Metallteil (1) Auflagensätze und/oder Einlagenzätze verschiedener Abmessungen zugeordnet sind.

5. Endoprothese nach einem der Ansprüche 1 bis 4 in Form einer Femurprothese, dadurch gekennzeichnet, daß der Prothesenschaft (1) als Flachprofil mit lateral angeordneten, vorzugsweise das Schaftprofil ausfüllenden, Platten aus Implantatmaterial ausgebildet ist.

6. Endoprothese nach einem der Ansprüche 1 bis 4 in Form einer Femurprothese, dadurch gekennzeichnet, daß der Prothesenschaft(1)in seinem distalen bereich bis zum Ende gespalten ist (5) und das Implantatmaterial als Einlageblock(2) in einer im wesentlichen Dreieckprofil aufweisenden Durchbrechung im proximalen Bereich des Prothesenschaftes vorgesehen ist (Fig. 2).

7. Endoprothese nach Anspruch 5 oder 6, dadurch gekennzeichnet, daß im Prothesenschaft (1) im Bereich des Trochanter maior mindestens eine Querbohrung (7) zur Muskelfixierung vorgesehen ist.

8. Endoprothese nach einem der Ansprüche 5 bis 7, dadurch gekennzeichnet, daß im Proximalbereich des Prothesenschaftes (1) eine Gewindebohrung (4)in Schaftrichtung zum Einschrauben des Prothesensetzkopfes vorgesehen ist.

9. Endoprothese nach einem der Ansprüche 5 bis 8, dadurch gekennzeichnet, daß im Proximalbereich des Prothesenschaftes eine Ausnehmung zum Einsetzen des jeweils benötigten Gelenkkopfes (6) vorgesehen ist.

10. Endoprothese nach einem der Ansprüche 5 bis 9, dadurch gekennzeichnet, daß zumindest der Prothesenschaft (1),vorzugsweise auch der Schulter/Halsbereich (3) der Prothese, räumlich gekrümmt ist, wobei insbesondere der Schulter/Halsbereich im Winkel zum Prothesenschaft steht.

*Fig. 1*

*Fig. 2*

*Fig. 3*

_Fig. 4_

**Europäisches**
**Patentamt**

# EUROPÄISCHER RECHERCHENBERICHT

Nummer der Anmeldung

EP    91 89 0194

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-0 337 757 (NOVAMED)<br>* Spalte 1, Zeile 46 - Spalte 2, Zeile 7 *<br>* Spalte 4, Zeile 9 - Zeile 31 *<br>* Spalte 6, Zeile 4 - Spalte 7, Zeile 14;<br>Abbildungen 1,12-15 *<br>--- | 1-3 | A61F2/30 |
| X | WO-A-8 706 842 (ERNST LEITZ WETZLAR GMBH)<br>* Seite 3, Zeile 24 - Zeile 18; Abbildungen 6-8,11 *<br>--- | 1,2,4 | |
| X | WO-A-8 505 027 (FRIED KRUPP GESELLSCHAFT MIT BESCHRÄNKTER HAFTUNG)<br>* Zusammenfassung; Ansprüche 1,3,10,13;<br>Abbildung 1 *<br>--- | 1 | |
| A | SOVIET PATENTS ABSTRACTS<br>Section PQ, Week 4787, 13. Januar 1988<br>Derwent Publications Ltd., London, GB;<br>Class P34, AN 87-332997<br>& SU-A-1 170 663 (MED TECH RES INST) 23. April 1987<br>* Zusammenfassung *<br>--- | 1 | |
| P,X | EP-A-0 417 330 (VSESOJUZNY NAUCHO-ISSLEDOVATELSKY I.M.T.)<br>* das ganze Dokument *<br>----- | 1 | RECHERCHIERTE SACHGEBIETE (Int. Cl.5 )<br><br>A61F |

Der vorliegende Recherchenbericht wurde für alle Patentansprüche erstellt

| Recherchenort | Abschlußdatum der Recherche | Prüfer |
|---|---|---|
| DEN HAAG | 18 DEZEMBER 1991 | GIMENEZ BURGOS R. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.........................................................................
& : Mitglied der gleichen Patentfamilie, übereinstimmendes
    Dokument

EPO FORM 1503 03.82 (P0403)